(19) **Europäisches Patentamt / European Patent Office / Office européen des brevets**

(11) **EP 2 251 013 A2**

(12) # EUROPEAN PATENT APPLICATION

(43) Date of publication:
17.11.2010 Bulletin 2010/46

(51) Int Cl.:
*A61K 31/66* (2006.01)  *A61L 9/04* (2006.01)
*A61K 8/46* (2006.01)  *A01N 57/00* (2006.01)
*A61P 35/00* (2006.01)

(21) Application number: 10008513.3

(22) Date of filing: 02.04.2007

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK RS

(30) Priority: 31.03.2006 US 396338

(62) Document number(s) of the earlier application(s) in accordance with Art. 76 EPC:
07754611.7 / 2 012 798

(71) Applicant: OxyPlus, Inc.
Brighton MA 02135 (US)

(72) Inventors:
• Nicolau, Claude
Newton
Massacgusetts 02459 (US)

• Greferath, Ruth
77694 Kehl (DE)
• Fylaktakidou, Konstantina C
55438 Ag. Pavlos
Thessaloniki (GR)
• Lehn, Jean-Marie
67000 Strasbourg (FR)

(74) Representative: Simpson, Mark Geoffrey
Potter Clarkson LLP
Park View House
58 The Ropewalk
Nottingham
NG1 5DD (GB)

Remarks:
This application was filed on 16-08-2010 as a divisional application to the application mentioned under INID code 62.

(54) **Use of inositol-tripyrophosphate in treating Alzheimer's disease, stroke or osteoporosis**

(57)    Inositol-tripyrophosphate is an allosteric effector of hemoglobin due to its ability to cross the plasma membrane of red blood cells and deliver oxygen to solid tumours, by lowering the oxygen affinity of the hemoglobin of red blood cells. The present invention is directed to the use of inositol-tripyrophosphate to reduce hemoglobin's affinity for oxygen in circulating red blood cells. The present invention is further directed to the use of inositol-tripyrophosphate for the treatment of Alzheimer's disease, stroke and osteoporosis.

Figure 1

EP 2 251 013 A2

**Description**

FIELD OF THE INVENTION

The present invention is directed to compositions and methods for using inositol-tripyrophosphate (ITPP) to enhance oxygen delivery by red blood. ITPP is an allosteric effector of hemoglobin which has the ability to cross the plasma membrane of red blood cells and lower the oxygen affinity of the hemoglobin of red blood cells. The present invention is further directed to the use of ITPP to inhibit angiogenesis and enhance radiation sensitivity of hypoxic tumors. The present invention is further directed to the use of ITPP to treat Alzheimer's disease.

BACKGROUND OF THE INVENTION

In the vascular system of an adult human being, blood has a volume of about 5 to 6 liters. Approximately one half of this volume is occupied by cells, including red blood cells (erythrocytes), white blood cells (leukocytes), and blood platelets. Red blood cells comprise the majority of the cellular components of blood. Plasma, the liquid portion of blood, is approximately 90 percent water and 10 percent various solutes. These solutes include plasma proteins, organic metabolites and waste products, and inorganic compounds.

The major function of red blood cells is to transport oxygen from the lungs to the tissues of the body, and transport carbon dioxide from the tissues to the lungs for removal. Very little oxygen is transported by the blood plasma because oxygen is only sparingly soluble in aqueous solutions. Most of the oxygen carried by the blood is transported by the hemoglobin of the erythrocytes. Erythrocytes in mammals do not contain nuclei, mitochondria or any other intracellular organelles, and they do not use oxygen in their own metabolism. Red blood cells contain about 35 percent by weight hemoglobin, which is responsible for binding and transporting oxygen.

Hemoglobin is a protein having a molecular weight of approximately 64,500 daltons. It contains four polypeptide chains and four heme prosthetic groups in which iron atoms are bound in the ferrous state. Normal globin, the protein portion of the hemoglobin molecule, consists of two alpha chains and two beta chains. Each of the four chains has a characteristic tertiary structure in which the chain is folded. The four polypeptide chains fit together in an approximately tetrahedral arrangement, to constitute the characteristic quaternary structure of hemoglobin. There is one heme group bound to each polypeptide chain which can reversibly bind one molecule of molecular oxygen. When hemoglobin combines with oxygen, oxyhemoglobin is formed. When oxygen is released, the oxyhemoglobin is reduced to deoxyhemoglobin.

Delivery of oxygen to tissues, including tumors, depends upon a number of factors including, but not limited to, the volume of blood flow, the number of red blood cells, the concentration of hemoglobin in the red blood cells, the oxygen affinity of the hemoglobin and, in certain species, on the molar ratio of intraerythrocytic hemoglobins with high and low oxygen affinity. The oxygen affinity of hemoglobin depends on four factors as well, namely: (1) the partial pressure of oxygen; (2) the pH; (3) the concentration of 2,3-diphosphoglycerate (DPG) in the hemoglobin; and (4) the concentration of carbon dioxide. In the lungs, at an oxygen partial pressure of 100 mm Hg, approximately 98% of circulating hemoglobin is saturated with oxygen. This represents the total oxygen transport capacity of the blood. When fully oxygenated, 100 ml of whole mammalian blood can carry about 21 ml of gaseous oxygen.

The effect of the partial pressure of oxygen and the pH on the ability of hemoglobin to bind oxygen is best illustrated by examination of the oxygen saturation curve of hemoglobin. An oxygen saturation curve plots the percentage of total oxygen-binding sites of a hemoglobin molecule that are occupied by oxygen molecules when solutions of the hemoglobin molecule are in equilibrium with different partial pressures of oxygen in the gas phase.

The oxygen saturation curve for hemoglobin is sigmoid. Thus, binding the first molecule of oxygen increases the affinity of the remaining hemoglobin for binding additional oxygen molecules. As the partial pressure of oxygen is increased, a plateau is approached at which each of the hemoglobin molecules is saturated and contains the upper limit of four molecules of oxygen.

The reversible binding of oxygen by hemoglobin is accompanied by the release of protons, according to the equation:

$$HHb^+ + O_2 \rightleftharpoons HbO_2 + H^+$$

Thus, an increase in the pH will pull the equilibrium to the right and cause hemoglobin to bind more oxygen at a given partial pressure. A decrease in the pH will decrease the amount of oxygen bound.

In the lungs, the partial pressure of oxygen in the air spaces is approximately 90 to 100 mm Hg and the pH is also high relative to normal blood pH (up to 7.6). Therefore, hemoglobin will tend to become almost maximally saturated with oxygen in the lungs. At that pressure and pH, hemoglobin is approximately 98 percent saturated with oxygen. On the other hand, in the capillaries in the interior of the peripheral tissues, the partial pressure of oxygen is only about 25 to 40 mm Hg and the pH is also nearly neutral (about 7.2 to 7.3). Because muscle cells use oxygen at a high rate, thereby lowering the local concentration of oxygen, the release of some of the bound oxygen to the tissue is favored. As the blood passes through the capillaries in the muscles, oxygen will be released from the nearly saturated hemoglobin in the red blood cells into the blood plasma and thence into the muscle cells. Hemoglobin will release about a fourth of its bound oxygen as it passes through the muscle capillaries, so that when it leaves the muscle, it will be only about 75 percent saturated. In general, the hemoglobin in the venous blood leaving the tissue cycles between about 65 and 97 percent saturation with oxygen in its repeated circuits between the lungs and the peripheral tissues. Thus, oxygen partial pressure and pH function together to effect the release of oxygen by hemoglobin.

A third important factor in regulating the degree of oxygenation of hemoglobin is the allosteric effector 2,3-diphosphoglycerate (DPG). DPG is the normal physiological effector of hemoglobin in mammalian erythrocytes. DPG regulates the oxygen-binding affinity of hemoglobin in the red blood cells in relationship to the oxygen partial pressure in the lungs. The higher the concentration of DPG in the cell, the lower the affinity of hemoglobin for oxygen.

When the delivery of oxygen to the tissues is chronically reduced, the concentration of DPG in the erythrocytes is higher than in normal individuals. For example, at high altitudes the partial pressure of oxygen is significantly less. Correspondingly, the partial pressure of oxygen in the tissues is less. Within a few hours after a normal human subject moves to a higher altitude, the DPG level in the red blood cells increases, causing more DPG to be bound and the oxygen affinity of the hemoglobin to decrease. Increases in the DPG level of red cells also occur in patients suffering from hypoxia. This adjustment allows the hemoglobin to release its bound oxygen more readily to the tissues to compensate for the decreased oxygenation of hemoglobin in the lungs. The reverse change occurs when people are acclimated to high altitudes and descend to lower altitudes.

As normally isolated from blood, hemoglobin contains a considerable amount of DPG. When hemoglobin is "stripped" of its DPG, it shows a much higher affinity for oxygen. When DPG is increased, the oxygen binding affinity of hemoglobin decreases. A physiologic allosteric effector such as DPG is therefore essential for the normal release of oxygen from hemoglobin in the tissues.

While DPG is the normal physiologic effector of hemoglobin in mammalian red blood cells, phosphorylated inositols are found to play the same role in the erythrocytes of some birds and reptiles. Although inositol hexaphosphate (IHP) is unable to pass through the mammalian erythrocyte membrane, it is capable of combining with hemoglobin of mammalian red blood cells at the binding site of DPG to modify the allosteric conformation of hemoglobin, the effect of which is to reduce the affinity of hemoglobin for oxygen. For example, DPG can be replaced by IHP, which is far more potent than DPG in reducing the oxygen affinity of hemoglobin. IHP has a 1000-fold higher affinity to hemoglobin than DPG (R. E. Benesch et al., Biochemistry, Vol. 16, pages 2594-2597(1977)) and increases the $P_{50}$ of hemoglobin up to values of 96.4 mm, Hg at pH 7.4, and 37 degrees C. (J. Biol. Chem., Vol. 250, pages 7093-7098(1975)).

The oxygen release capacity of mammalian red blood cells can be enhanced by introducing certain allosteric effectors of hemoglobin into erythrocytes, thereby decreasing the affinity of hemoglobin for oxygen and improving the oxygen economy of the blood. This phenomenon suggests various medical applications for treating individuals who are experiencing lowered oxygenation of their tissues due to the inadequate function of their lungs or circulatory system.

Because of the potential medical benefits to be achieved from the use of these modified erythrocytes, various techniques have been developed in the prior art to enable the encapsulation of allosteric effectors of hemoglobin in erythrocytes. Accordingly, numerous devices have been designed to assist or simplify the encapsulation procedure. The encapsulation methods known in the art include osmotic pulse (swelling) and reconstitution of cells, controlled lysis and reseating, incorporation of liposomes, and electroporation. Current methods of electroporation make the procedure commercially impractical on a scale suitable for commercial use.

The following references describe the incorporation of polyphosphates into red blood cells by the interaction of liposomes loaded with IHP: Gersonde, et al., "Modification of the Oxygen Affinity of Intracellular Haemoglobin by Incorporation of Polyphosphates into Intact Red Blood Cells and Enhanced 02 Release in the Capillary System", Biblthca. Haemat, No. 46, pp. 81-92(1980); Gersonde, et al., "Enhancement of the O2 Release Capacity and of the Bohr-Effect of Human Red Blood Cells after Incorporation of Inositol Hexaphosphate by Fusion with Effector-Containing Lipid Vesicles", Origins of Cooperative Binding of Hemoglobin, (1982); and Weiner, "Right Shifting of Hb-O2 Dissociation in Viable Red Cells by Liposomal Technique," Biology of the Cell, Vol. 47, (1983).

Additionally, U.S. Pat. Nos. 4,192,869, 4,321,259, and 4,473,563 to Nicolau et al. describe a method whereby fluid-charged lipid vesicles are fused with erythrocyte membranes, depositing their contents into the red blood cells. In this manner it is possible to transport allosteric effectors such as IHP into erythrocytes, where, due to its much higher binding constant IHP replaces DPG at its binding site in hemoglobin.

In accordance with the liposome technique, IHP is dissolved in a phosphate buffer until the solution is saturated and a mixture of lipid vesicles is suspended in the solution. The suspension is then subjected to ultrasonic treatment or an injection process, and then centrifuged. The upper suspension contains small lipid vesicles containing IHP, which are then collected. Erythrocytes are added to the collected suspension and incubated, during which time the lipid vesicles containing IHP fuse with the cell membranes of the erythrocytes, thereby depositing their contents into the interior of the erythrocyte. The modified erythrocytes are then washed and added to plasma to complete the product.

The drawbacks associated with the liposomal technique include poor reproducibility of the IHP concentrations incorporated in the red blood cells and significant hemolysis of the red blood cells following treatment. Additionally, commercialization is not practical because the procedure is tedious and complicated.

In an attempt to solve the drawbacks associated with the liposomal technique, a method of lysing and the resealing red blood cells was developed. This method is described in the following publication: Nicolau, et al., "Incorporation of Allosteric Effectors of Hemoglobin in Red Blood Cells. Physiologic Effects," Biblthca. Haemat., No. 51, pp. 92-107, (1985). Related U.S. Pat. Nos. 4,752,586 and 4,652,449 to Ropars et al. also describe a procedure of encapsulating substances having biological activity in human or animal erythrocytes by controlled lysis and resealing of the erythrocytes, which avoids the red blood cell-liposome interactions.

The technique is best characterized as a continuous flow dialysis system which functions in a manner similar to the osmotic pulse technique. Specifically, the primary compartment of at least one dialysis element is continuously supplied with an aqueous suspension of erythrocytes while the secondary compartment of the dialysis element contains an aqueous solution which is hypotonic with respect to the erythrocyte suspension. The hypotonic solution causes the erythrocytes to lyse. The erythrocyte lysate is then contacted with the biologically active substance to be incorporated into the erythrocyte. To reseal the membranes of the erythrocytes, the osmotic and/or oncotic pressure of the erythrocyte lysate is increased and the suspension of resealed erythrocytes is recovered.

In related U.S. Pat. Nos. 4,874,690 and 5,043,261 to Goodrich et al., a related technique involving lyophilization and reconstitution of red blood cells is disclosed. As part of the process of reconstituting the red blood cells, the addition of various polyanions, including IHP, is described. Treatment of the red blood cells according to the process disclosed results in a cell with unaffected activity. Presumably, the IHP is incorporated into the cell during the reconstitution process, thereby maintaining the activity of the hemoglobin.

In U.S. Pat. Nos. 4,478,824 and 4,931,276 to Franco et al., a second related method and apparatus is described for introducing effectively non-ionic agents, including IHP, into mammalian red blood cells by effectively lysing and resealing the cells. The procedure is described as the "osmotic pulse technique." In practicing the osmotic pulse technique, a supply of packed red blood cells is suspended and incubated in a solution containing a compound which readily diffuses into and out of the cells, the concentration of the compound being sufficient to cause diffusion thereof into the cells so that the contents of the cells become hypertonic. Next, a transmembrane ionic gradient is created by diluting the solution containing the hypertonic cells with an essentially isotonic aqueous medium in the presence of at least one desired agent to be introduced, thereby causing diffusion of water into the cells with a consequent swelling and an increases in permeability of the outer membranes of the cells. This "osmotic pulse" causes the diffusion of water into the cells and a resultant swelling of the cells which increase the permeability of the outer cell membrane to the desired agent. The increase in permeability of the membrane is maintained for a period of time sufficient only to permit transport of least one agent into the cells and diffusion of the compound out of the cells.

Polyanions which may be used in practicing the osmotic pulse technique include pyrophosphate, tripolyphosphate, phosphorylated inositols, 2,3-diphosphoglycerate (DPG), adenosine triphosphate, heparin, and polycarboxylic acids which are watersoluble, and non-disruptive to the lipid outer bilayer membranes of red blood cells.

The osmotic pulse technique has several shortcomings including low yield of encapsulation, incomplete resealing, lose of cell content and a corresponding decrease in the life span of the cells. The technique is tedious, complicated and unsuited to automation. For these reasons, the osmotic pulse technique has had little commercial success.

Another method for encapsulating various biologically-active substances in erythrocytes is electroporation. Electroporation has been used for encapsulation of foreign molecules in different cell types, including IHP in red blood cells, as described in Mouneimne, et al., "Stable rightward shifts of the oxyhemoglobin dissociation curve induced by encapsulation of inositol hexaphosphate in red blood cells using electroporation," FEBS, Vol. 275, No. 1, 2, pp. 117-120 (1990) Also see U.S. Patent No. 5,612,207.

Angiogenesis is the generation of new blood vessels into a tissue or organ and is related to oxygen tension in the tissues. Under normal physiological conditions, humans and animals undergo angiogenesis only in very specific, restricted situations. For example, angiogenesis is normally observed in wound healing, fetal and embryonal development, and formation of the corpus luteum, endometrium and placenta.

Angiogenesis is controlled through a highly regulated system of angiogenic stimulators and inhibitors. The control of angiogenesis is altered in certain disease states and, in many cases, pathological damage associated with the diseases is related to uncontrolled angiogenesis. Both controlled and uncontrolled angiogenesis are thought to proceed in a similar manner. Endothelial cells and pericytes, surrounded by a basement membrane, form capillary blood vessels. Angiogenesis begins with the erosion of the basement membrane by enzymes released by endothelial cells and leukocytes. Endothelial cells, lining the lumen of blood vessels, then protrude through the basement membrane.

Angiogenic stimulants induce the endothelial cells to migrate through the eroded basement membrane. The migrating cells form a "sprout" off the parent blood vessel where the endothelial cells undergo mitosis and proliferate. The endothelial sprouts merge with each other to form capillary loops, creating a new blood vessel.

Persistent, unregulated angiogenesis occurs in many disease states, tumor metastases, and abnormal growth by endothelial cells. The diverse pathological disease states in which unregulated angiogenesis is present have been grouped together as angiogenic-dependent or angiogenic-associated diseases.

The hypothesis that tumor growth is angiogenesis-dependent was first proposed in 1971. (Folkman, New Eng. J. Med., 285:1182-86 (1971)). In its simplest terms, this hypothesis states: "Once tumor 'take' has occurred, every increase in tumor cell population must be preceded by an increase in new capillaries converging on the tumor." Tumor 'take' is currently understood to indicate a prevascular phase of tumor growth in which a population of tumor cells occupying a few cubic millimeters volume, and not exceeding a few million cells, can survive on existing host microvessels. Expansion of tumor volume beyond this phase requires the induction of new capillary blood vessels. For example, pulmonary micrometastases in the early prevascular phase in mice would be undetectable except by high power microscopy on histological sections.

Angiogenesis has been associated with a number of different types of cancer, including solid tumors and blood-borne tumors. Solid tumors with which angiogenesis has been associated include, but are not limited to, rhabdomyosarcomas, retinoblastoma, Ewing's sarcoma, neuroblastoma, and osteosarcoma. Angiogenesis is also associated with blood-borne tumors, such as leukemias, any of various acute or chronic neoplastic diseases of the bone marrow in which unrestrained proliferation of white blood cells occurs, usually accompanied by anemia, impaired blood clotting, and enlargement of the lymph nodes, liver and spleen. It is believed that angiogenesis plays a role in the abnormalities in the bone marrow that give rise to leukemia tumours and multiple myeloma diseases.

One of the most frequent angiogenic diseases of childhood is the hemangioma. A hemangioma is a tumor composed of newly formed blood vessels. In most cases the tumors are benign and regress without intervention. In more severe cases, the tumors progress to large cavernous and infiltrative forms and create clinical complications. Systemic forms of hemangiomas, hemangiomatoses, have a high mortality rate. Therapy-resistant hemangiomas exist that cannot be treated with therapeutics currently in use.

Another angiogenesis associated disease is rheumatoid arthritis. The blood vessels in the synovial lining of the joints undergo angiogenesis. In addition to forming new vascular networks, the endothelial cells release factors and reactive oxygen species that lead to pannus growth and cartilage destruction. Angiogenesis may also play a role in osteoarthritis. The activation of the chondrocytes by angiogenic-related factors contributes to the destruction of the joint. At a later stage, the angiogenic factors promote new bone growth. Therapeutic intervention that prevents the cartilage destruction could halt the progress of the disease and provide relief for persons suffering with arthritis.

Chronic inflammation may also involve pathological angiogenesis. Such diseases as ulcerative colitis and Crohn's disease show histological changes with the ingrowth of new blood vessels into inflamed tissues. Bartonelosis, a bacterial infection found in South America, can result in a chronic stage that is characterized by proliferation of vascular endothelial cells. Another pathological role associated with angiogenesis is found in atherosclerosis. The plaques formed within the lumen of blood vessels have been shown to have angiogenic stimulatory activity.

As mentioned above, several lines of evidence indicate that angiogenesis is essential for the growth and persistence of solid tumors and their metastases. Once angiogenesis is stimulated, tumors upregulate the production of a variety of angiogenic factors, including fibroblast growth factors (aFGF and bFGF) and vascular endothelial growth factor/vascular permeability factor (VEGF/VPF) [2,3].

The role of VEGF in the regulation of angiogenesis has been the object of intense investigation [5-10]. Whereas VEGF represents a critical, rate-limiting step in physiological angiogenesis, it appears to be also important in pathological angiogenesis, such as that associated with tumor growth [11]. VEGF is also known as vascular permeability factor, based on its ability to induce vascular leakage [13]. Several solid tumors produce ample amounts of VEGF, which stimulates proliferation and migration of endothelial cells, thereby inducing neovascularization [12,13]. VEGF expression has been shown to significantly affect the prognosis of different kinds of human cancer. Oxygen tension in the tumor has a key role in regulating the expression of VEGF gene. VEGF mRNA expression is induced by exposure to low oxygen tension under a variety of pathophysiological circumstances [13]. Growing tumors are characterized by hypoxia, which induces expression of VEGF and may also be a predictive factor for the occurrence of metastatic disease.

Alzheimer's disease is a progressive, neurodegenerative disease characterized by amyloid plaques and neurofibrillary tangles composed of misplaced proteins in parts of the brain involved in memory, learning, language, and reasoning. Age is the most important risk factor for Alzheimer's disease and the number of people with the disease doubles every five years beyond age 65. As the disease progresses, communication between the neurons of the brain break down. In early stages, short-term memory begins to fail. Over time, Alzheimer's diseases destroys cognition, personality and the ability to function. Alzheimer's disease is the most common cause of dementia in older adults. Dementia is a loss of mental functions, that are severe enough to interfere with daily functioning.

There are two basic types of Alzheimer's disease: early on-set Alzheimer's disease tends to strike people under age 65 and is more likely to run in families. Late-onset Alzheimer's disease, the more common type, generally afflicts people after age 65. The pathological basis for Alzheimer's disease remains unknown, although researchers have made some progress. Suggested mechanisms for the disease include cerebral hypoperfusion, inflammation, gene polymorphisms, and molecular lesions in the brain. It is suspected that the vascular endothelial cell has a central role in the progressive destruction of cortical neurons in Alzheimer's disease.

In Alzheimer's disease, the brain endothelium secretes the precursor substrate for the beta-amyloid plaque and a neurotoxic peptide that selectively kills cortical neurons. Large populations of endothelial cells are activated by angiogenesis due to brain hypoxia and inflammation. Results of epidemiological studies have shown that long-term use of non-steroidal anti-inflammatory drugs, statins, histamine H2-receptor blockers, or calcium-channel blockers have an effect on Alzheimer's disease. This effect may be largely due to the ability of these drugs to inhibit angiogenesis. Research has demonstrated that Alzheimer's disease is an angiogenesis-dependent disorder. (Vagnucci, et al., Alzheimer's disease and angiogenesis. Lancet, 361 (9357): 605-608, Feb. 15, 2003.) Further, angiogenesis as the result of dysfunctional cerebral vasculature, may be both a consequence and contributory factor to the etiopathology of the Alzheimer's disease process. (Pogue, et al., Angiogenic signaling in Alzheimer's disease. Neuroreport, 15 (9): 1507-1510, June 28, 2004

What is needed, therefore, is a composition and method that is effective in treating Alzheimer's disease and other diseases characterized by abnormal angiogenesis. In addition, what is needed is a simple and easily administered, preferably orally, composition that is capable of causing significant right shifts of the $P_{50}$ value for red blood cells.

SUMMARY OF THE INVENTION

The present invention provides a composition comprising inositol-tripyrophosphate (ITPP) that is effective in treating diseases characterized by abnormal angiogenesis. The present invention also provides for methods of using ITPP for increasing the regulated delivery of oxygen to tissues including tumors. For example, the regulation of vascular endothelial growth factor (VEGF) in a human or animal can be effected using ITPP which has entered the red blood cell, thus lowering the affinity for oxygen of circulating erythrocytes. The effect of ITPP on VEGF mRNA expression, protein concentration, and tumor cell proliferation are possible with the present invention. Also, a method of regulating VEGF expression, both *in vitro* and *in vivo*, has been developed using ITPP.

The present invention further comprises compositions and methods for using ITPP in red blood cells to deliver oxygen to solid tumors, to inhibit angiogenesis and to enhance radiation sensitivity of hypoxic tumors. The present invention is further directed to the use of ITPP to enhance $PO_2$ in hypoxic tumors. ITPP, being an allosteric effector of hemoglobin, is capable of reducing hemoglobin's affinity for oxygen and enhances the release of oxygen by hemoglobin. Upon cellular demand, ITPP inhibits VEGF expression in tumor cells and, thus, angiogenesis.

The present invention further comprises compositions and methods for using ITPP in red blood cells to treat Alzheimer's disease.

A disease characterized by undesirable angiogenesis or undesirable angiogenesis, as defined herein includes, but is not limited to, excessive or abnormal stimulation of endothelial cells (e.g. atherosclerosis), blood borne tumors, solid tumors and tumor metastasis, benign tumors, for example, hemangiomas, acoustic neuromas, neurofibromas, trachomas, and pyogenic granulomas, vascular malfunctions and disorders, such as Alzheimer's disease, abnormal wound healing, inflammatory and immune disorders, Bechet's disease, gout, or gouty arthritis, diabetic retinopathy and other ocular angiogenic diseases such as retinopathy of prematurity (retrolental fibroplasic), macular degeneration, corneal graft rejection, neovascular glaucoma and Osler Weber syndrome (Osler-Weber-Rendu disease). Cancers that can be treated by the present invention include, but is not limited to, breast cancer, prostrate cancer, renal cell cancer, brain cancer, ovarian cancer, colon cancer, bladder cancer, pancreatic cancer, stomach cancer, esophageal cancer, cutaneous melanoma, liver cancer, lung cancer, testicular cancer, kidney cancer, bladder cancer, cervical cancer, lymphoma, parathyroid cancer, penile cancer, rectal cancer, small intestine cancer, thyroid cancer, uterine cancer, Hodgkin's lymphoma, lip and oral cancer, skin cancer, leukemia or multiple myeloma.

An object of the invention is to provide a composition and method for treating cancer and other angiogenic disease states and conditions.

Another object of the invention is to provide a composition and method for enhancing oxygen delivery to hypoxic tumors.

Yet another object of the invention is to provide a composition and method for inhibiting angiogenesis.

A further object of the invention is to provide a composition and method for enhancing radiation sensitivity of hypoxic tumors.

It is yet another object of the invention to provide a composition and method of treating hypoxic tumors and diseases.

Another object of the invention is to provide a composition and method that can regulate oxygen tension in the tissue, especially a tumor.

A further object of the invention is to provide a composition and method for treating Alzheimer's disease.

A further object of the invention is to provide a simple and easily administered, preferably orally, composition that is capable of causing significant right shifts of the $P_{50}$ value for red blood cells.

These and other objects, features and advantages of the present invention will become apparent after a review of the following detailed description of the disclosed embodiments.

BRIEF DESCRIPTION OF THE DRAWINGS

Figure 1 depicts the chemical structure of inositol-tri-pyrophosphate (ITPP).

Figure 2 shows the time course of the induced right shift of the $O_2$-hemoglobin dissociation curve (ODC) in the mice ingesting ITPP for 4 days as well as the absence of significant $P_{50}$ shifts in the control animals.

Figure 3 shows that the level of ions, such as sodium and potassium and calcium, were normal after oral application of ITPP in mice.

Figure 4 shows the relation of $P_{50}$ shift [%] to number of crythrocytes/$mm^3$ in mice having received ITPP.

Figure 5 shows the dose-effect curve of ITPP in mice injected i.p. 200$\mu$l of a 45-120 mM ITPP solution (= 0.17- 0.88 g ITPP /kg body weight).

Figure 6 shows an agarose gel indicating the VEGF mRNA concentrations in tumors from control and ITPP drinking animals.

Figure 7 shows the Western blot assay of the expressed VEGF in tumors of control and ITPP-treated Lewis Lung carcinoma (LLC) tumor-bearing animals.

DETAILED DESCRIPTION OF THE INVENTION

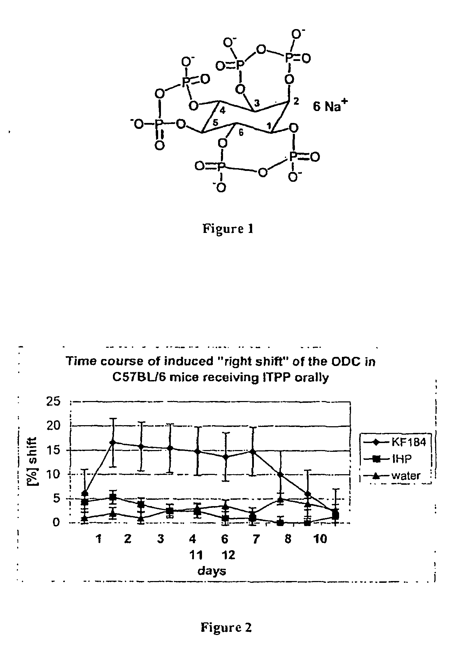

Compositions that are useful in accordance with the present invention include inositol-tripyrophosphate (ITPP) (Figure 1) and salts thereof like Na, Ca, $NH_4$ salts of ITPP, which exhibits anti-angiogenic and anti-tumor properties, and are useful in controlling angiogenesis-, or proliferation-related events, conditions or substances. As used herein, the control of an angiogenic-, or proliferation-related event, condition, or substance refers to any qualitative or quantitative change in any type of factor, condition, activity, indicator, chemical or combination of chemicals, mRNA, receptor, marker, mediator, protein, transcriptional activity or the like, that may be or is believed to be related to angiogenesis or proliferation, and that results from administering the composition of the present invention. Those skilled in the art will appreciate that the invention extends to other compositions or compounds in the claims below, having the described characteristics. These characteristics can be determined for each test compound using the assays detailed below and elsewhere in the literature.

Other such assays include counting of cells in tissue culture plates or assessment of cell number through metabolic assays or incorporation into DNA of labeled (radiochemically, for example $^3$H-thymidine, or fluorescently labeled) or immuno-reactive (BrdU) nucleotides. In addition, antiangiogenic activity may be evaluated through endothelial cell migration, endothelial cell tubule formation, or vessel outgrowth in *ex-vivo* models such as rat aortic rings.

When administered orally, ITPP exhibits anti-tumor and anti-proliferative activity with little or no toxicity. ITPP was tested for its ability to induce a decrease of the $O_2$-affinity of hemoglobin measured as a shift of the $P_{50}$ value ($P_{50}$ at 50% saturation of hemoglobin). With murine hemoglobin and whole blood, $P_{50}$ shifts to higher $PO_2$ of up to 250% with hemoglobin and up to 40% with whole blood were observed.

The results obtained with ITPP in mice and pigs strongly suggest the possibility of its development as a therapeutic, due to its ability to enhance, in a regulated manner, oxygen delivery by red blood cells in the cases of blood flow impairment.

ITPP, when administered orally, intravenously, or intraperitoneally, inhibits angiogenesis in growing tumors by enhancing $PO_2$ in the forming tumors. This invention further provides for methods of regulation of vascular endothelial growth factor (VEGF) in a human or animal, by administering to the human or animal an effective amount of ITPP. More particularly, this invention provides for dose-dependent effects of ITPP on VEGF mRNA and protein expressions in the LLC cell line. VEGF gene expression in tumor bearing C57BL/6 mice was assayed and the effects of ITPP-induced down regulation of VEGF have been determined and correlated with modulation of cell proliferation. This invention resulted in the development of methods to control VEGF mRNA expression, protein concentration, and tumor cell proliferation. The results of these studies indicate a strong correlation between dose-dependent TTPP-induced down regulation of VEGF and cellular proliferation and suggests that ITPP can reduce VEGF mediated tumor angiogenesis, as well as the rate of tumor cell proliferation. Thus, down-regulation of VEGF by ITPP decreases tumor cell proliferation.

The shifting of the $P_{50}$ value to higher $O_2$-partial pressures inhibits the expression of the hypoxia gene encoding VEGF in the tumors. Expression of the hypoxia gene encoding VEGF is necessary for angiogenesis to be stimulated in tumors. If this does not occur, angiogenesis is seriously inhibited and new vessels are not formed in tumors.

The results obtained concerning VEGF expression suggests that oxygen partial pressure in tumors is elevated upon administration of ITPP, as this elevation is the cause of inhibition of expression of this hypoxia gene. This observation raises a very important question, namely whether this enhancement of $PO_2$ may not act as a powerful radiosensitizer of cancer cells. Oxygen is a very potent radiosensitizer and, if indeed $PO_2$ in the tumors is enhanced by ITPP, this may have major consequences in enhancing the efficacy of radiation therapy of cancer.

ITPP is a potential significant adjuvant in the therapy of solid tumors as inhibitor of angiogenesis on one hand, and as radiosensitizer on the other.

Alzheimer's disease is linked to angiogenesis under the hypothesis that the vascular endothelial cells have a central role in the progressive destruction of cortical neurons. This invention provides for ITPP-induced down regulation of vascular endothelial growth factor (VEGF) and cellular proliferation and suggests that ITPP can reduce VEGF mediated Alzheimer's disease. This invention contemplates regulating VEGF in a human or animal, by administering to the human or animal an effective amount of ITPP. This invention resulted in the development of methods to control VEGF mRNA expression, protein concentration, and endothelial cell proliferation.

It is known that medial temporal oxygen metabolism is markedly affected in patients with mild-to-moderate Alzheimer's disease. This measure substantiated the functional impairment of the medial temporal region in Alzheimer's disease. It also known that mean oxygen metabolism in the medial temporal, as well as in the parietal and lateral temporal cortices is significantly lower in the patients that are shown to have Alzheimer's disease than in control groups without Alzheimer's disease (see Ishii et al., J. Nucl. Med. 37(7):1159-65, Jul 1996, which is herein incorporated by reference in its entirety). Thus, one potential means of treating patients shown to have Alzheimer's disease is to increase oxygen across the blood brain barrier. One method of doing so would be to use an allosteric effector of hemoglobin such as treatment with ITPP.

The use of ITPP may also help in the treatment of a variety of vascular diseases associated with various forms of dementia. Because the brain relies on a network of vessels to bring it oxygen-bearing blood, if the oxygen supply to the brain fails, brain cells are likely to die and this can cause symptoms of vascular dementia. These symptoms can occur either suddenly, following a stroke, or over time through a series of small strokes. Thus, one potential means of treating patients with vascular diseases associated with various forms of dementia is to increase the oxygen available to affected areas such as across the blood brain barrier. One method of doing so would be to use an allosteric effector of hemoglobin such as treatment with ITPP.

Moreover, treatment of an individual with an allosteric effector of hemoglobin may have beneficial effects for both stroke victims and osteoporosis. Although stroke and the bone-thinning disease osteoporosis are usually thought of as two distinct health problems, it has been found that there may be a connection between them. Patients who survive strokes are significantly more likely to suffer from osteoporosis, a disease that puts them at high risk for bone fractures. Often, the fractures in stroke patients occur on the side of the body that has been paralyzed from the stroke.

It is known that a stroke occurs when the supply of blood and oxygen to the brain ceases or is greatly reduced. If a portion of the brain loses its supply of nutrient-rich blood and oxygen, the bodily functions controlled by that part of the brain (vision, speech, walking, etc.) are impaired. Annually, more than 500,000 people in the United States suffer strokes and 150,000 of those people die as a result thereof. One means of increasing oxygen flow to the brain is by use of an allosteric effector of hemoglobin such as treatment with ITPP. Accordingly, a potential method of treating individuals who might potentially suffer stroke or osteoporosis is by treatment of an individual with, for example, ITPP.

Also contemplated by the present invention are implants or other devices comprised of the compounds or drugs of ITPP or prodrugs thereof where the drug or prodrug is formulated in a biodegradable or non-biodegradable polymer for sustained release. Non-biodegradable polymers release the drug in a controlled fashion through physical or mechanical processes without the polymer itself being degraded. Biodegradable polymers are designed to gradually be hydrolyzed or solubilized by natural processes in the body, allowing gradual release of the admixed drug or prodrug. The drug or prodrug can be chemically linked to the polymer or can be incorporated into the polymer by admixture. Both biodegradable and non-biodegradable polymers and the process by which drugs are incorporated into the polymers for controlled release are well known to those skilled in the art. Examples of such polymers can be found in many references, such as Brem et al., J. Neurosurg 74: pp. 441-446 (1991). These implants or devices can be implanted in the vicinity where delivery is desired, for example, at the site of a tumor.

In addition to the compounds of the present invention, the pharmaceutical composition of this invention may also contain, or be co-administered (simultaneously or sequentially) with, one or more pharmacological agents of value in treating one or more disease conditions referred to hereinabove.

A person skilled in the art will be able by reference to standard texts, such as Remington's Pharmaceutical Sciences 17th edition, to determine how the formulations are to be made and how these may be administered.

In a further aspect of the present invention there is provided use of compounds of ITPP or prodrugs thereof according to the present invention for the preparation of a medicament for the prophylaxis or treatment of conditions associated with angiogenesis or accelerated cell division or inflammation.

In another embodiment, the composition of the present invention can be used to treat any of Alzheimer's disease, stroke, and/or osteoporosis by delivering an effective amount of TTPP.

In a further aspect of the present invention there is provided a pharmaceutical composition comprising compounds of ITPP or prodrugs thereof according to the present invention, together with a pharmaceutically acceptable carrier, diluent or excipient

The pharmaceutical composition may be used for the prophylaxis or treatment of conditions associated with angiogenesis or accelerated cell division or inflammation.

In a still further aspect of the present invention there is provided a method of prophylaxis or treatment of a condition associated with angiogenesis or accelerated or increased amounts of cell division hypertrophic growth or inflammation, said method including administering to a patient in need of such prophylaxis or treatment an effective amount of compounds of ITPP or prodrugs thereof according to the present invention, as described herein. It should be understood that prophylaxis or treatment of said condition includes amelioration of said condition.

By "an effective amount" is meant a therapeutically or prophylactically effective amount. Such amounts can be readily determined by an appropriately skilled person, taking into account the condition to be treated, the route of administration and other relevant factors. Such a person will readily be able to determine a suitable dose, mode and frequency of administration.

The compositions described above can be provided as physiologically acceptable formulations using known techniques, and these formulations can be administered by standard routes. In general, the combinations may be administered by the topical, oral, rectal, intraperitoneal or parenteral (*e.g.*, intravenous, subcutaneous or intramuscular) route. In addition, the combinations may be incorporated into polymers allowing for sustained release, the polymers being implanted in the vicinity of where delivery is desired, for example, at the site of a tumor. The dosage of the composition will depend on the condition being treated, the particular derivative used, and other clinical factors such as weight and condition of the patient and the route of administration of the compound. However, for oral administration, a recommended dosage is in the range of 0.1 to 5.0 g/kg/day. A preferred dosage for oral administration is in the range of 0.5 to 2.0 g/kg/day. An especially preferred dosage for oral administration is in the range of 0.80 to 1.0 g/kg/day.

The formulations in accordance with the present invention can be administered in the form of tablet, a capsule, a lozenge, a cachet, a solution, a suspension, an emulsion, a powder, an aerosol, a suppository, a spray, a pastille, an ointment, a cream, a paste, a foam, a gel, a tampon, a pessary, a granule, a bolus, a mouthwash, or a transdermal patch.

The formulations include those suitable for oral, rectal, nasal, inhalation, topical (including dermal, transdermal, buccal and sublingual), vaginal, parenteral (including subcutaneous, intramuscular, intravenous, intraperitoneal, intradermal, intraocular, intratracheal, and epidural) or inhalation administration. The formulations may conveniently be presented in unit dosage form and may be prepared by conventional pharmaceutical techniques. Such techniques include the step of bringing into association the active ingredient and a pharmaceutical carrier(s) or excipient(s). In general, the formulations are prepared by uniformly and intimately bringing into association the active ingredient with liquid carriers or finely divided solid carriers or both, and then, if necessary, shaping the product

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil emulsion, etc.

A tablet may be made by compression or molding, optionally with one or more accessory ingredients. Compressed tablets may be prepared by compressing, in a suitable machine, the active ingredient in a free-flowing form such as a powder or granules, optionally mixed with a binder, lubricant, inert diluent, preservative, surface-active or dispersing agent. Molded tablets may be made by molding, in a suitable machine, a mixture of the powdered compound moistened with an inert liquid diluent. The tablets may optionally be coated or scored and may be formulated so as to provide a slow or controlled release of the active ingredient therein.

Formulations suitable for topical administration in the mouth include lozenges comprising the ingredients in a flavored basis, usually sucrose and acacia or tragacanth; pastilles comprising the active ingredient in an inert basis such as gelatin and glycerin, or sucrose and acacia; and mouthwashes comprising the ingredient to be administered in a suitable liquid carrier.

Formulations suitable for topical administration to the skin may be presented as ointments, creams, gels and pastes comprising the ingredient to be administered in a pharmaceutical acceptable carrier. A preferred topical delivery system is a transdermal patch containing the ingredient to be administered.

Formulations for rectal administration may be presented as a suppository with a suitable base comprising, for example, cocoa butter or a salicylate.

Formulations suitable for nasal administration, wherein the carrier is a solid, include a coarse powder having a particle size, for example, in the range of 20 to 500 microns which is administered in the manner in which snuff is taken; *i.e.*, by rapid inhalation through the nasal passage from a container of the powder held close up to the nose. Suitable formulations, wherein the carrier is a liquid, for administration, as for example, a nasal spray or as nasal drops, include aqueous or oily solutions of the active ingredient.

Formulations suitable for vaginal administration may be presented as pessaries, tampons, creams, gels, pastes, foams or spray formulations containing, in addition to the active ingredient, ingredients such as carriers as are known in the art to be appropriate.

Formulation suitable for inhalation may be presented as mists, dusts, powders or spray formulations containing, in addition to the active ingredient, ingredients such as carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in freeze-dried (lyophilized) conditions requiring only the addition of a sterile liquid carrier, for example, water for injections, immediately prior to use. Extemporaneous injection solutions and suspensions may be prepared from sterile powders, granules and tablets of the kinds previously described.

Preferred unit dosage formulations are those containing a daily dose or unit, daily sub-dose, as herein above recited, or an appropriate fraction thereof, of the administered ingredient.

It should be understood that in addition to the ingredients, particularly mentioned above, the formulations of the present invention may include other agents conventional in the art having regard to the type of formulation in question, for example, those suitable for oral administration may include flavoring agents.

For the *in vitro* experiments ITPP was dissolved in deionized water, pH was adjusted at pH 7 and, for incubation with whole blood, the osmolarity of the ITPP solutions was adjusted with glucose to 270-297 mOsM. Mixtures of hemoglobin and ITPP were measured with a HEMOX analyzer (PD Marketing, London) immediately. Red blood cells were incubated with ITPP for 1 hour at 37°C. Following incubation, the cells were washed 3 times with Bis-Tris-buffer (PH=7.0) and then used for $P_{50}$ measurement

In experiments conducted *in vivo*, in which ITPP was administered orally, a significant shift of the $P_{50}$ value of circulating RBCs was observed. ITPP was dissolved in drinking water at a 20g/L-concentration (= 27mM, pH ~7.0.) and offered for drinking ad libitum.

The following examples illustrate but not limit the invention. Thus, the examples are presented with the understanding that modifications may be made and still be within the spirit of the invention.

EXAMPLE 1

Induced Right Shift of the $O_2$-Hemoglobin Dissociation Curve (ODC) in Mice (Orally Administered)

Twelve (12) C57BL/6 mice were fed the ITPP-solution (20g/L-concentration = 27mM, pH ~7.0) for 4 days (up to 25 ml per 24 hrs). Three (3) control mice drank pure water, and four (4 ) control mice a solution of myo-inositol hexaphosphate (IHP) (same concentration and pH as ITPP). Blood was collected from all mice on day 0 (before treatment started), and on days 1, 2, 4, 6, 7, 8, 10, 11 and 12 (after treatment had started), in order to measure $P_{50}$ values.

*Results*

ITPP was neither rejected by the mice, nor harmful to the animals.

Oral application of ITPP caused significant right shifts of Pso (up to 31%) in mice.

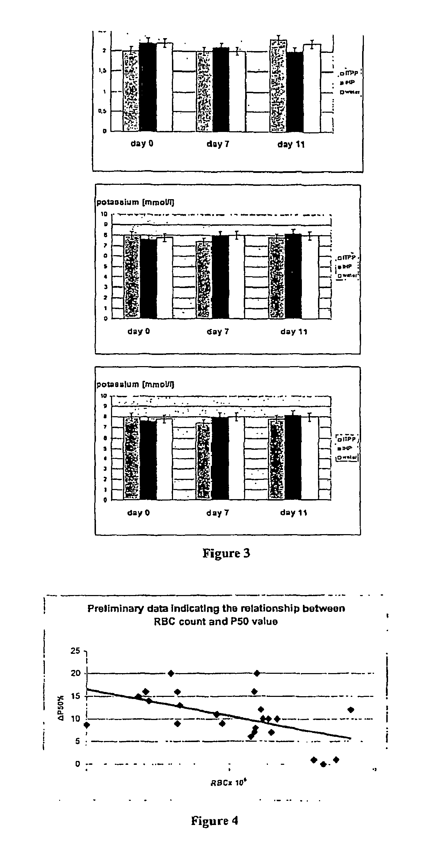

ITPP, when orally administered at a concentration of 27 mM, causes a right shift of the $P_{50}$ value in murine circulating red blood cells (Figure 2). There is a time lag of approximately 48 hrs before the maximum shift is attained. Maximal $P_{50}$ shifts are reached between day 2 and day 4, after beginning oral administration of ITPP. After 12 days, $P_{50}$ values are back to control values, when ingestion is stopped on day 4. There is a significant effect of ITPP ingestion on the number of red blood cells. Although not wishing to be bound by theory, it is believed that effect of ITPP ingestion on the number of red blood cells (down-regulation of erythropoiesis) is due to the increased $P_{50}$. Hemolysis can be ruled out, as lysis of the red blood cells never occurred *in vitro.* The level of ions, such as sodium and potassium and calcium were normal after oral application of ITPP in mice (Figure 3). Figure 3 contains the mean values and SD for the serum concentration of sodium, potassium and calcium obtained on day 0, 7 and 11 after start drinking ITPP (4 mice), IHP (3 mice) or water (3 mice).

Blood counts were measured from all mice, on day 0, 7 and 11. The number of red blood cells in mice having ingested ITPP was reduced. There were no significant differences in the number of white blood cells (e.g. granulocytes, macrophages etc.) in blood from mice in different groups. Figure 4 shows the RBC counts for mice with shifted ODC as compared to controls. Figure 4 further shows the relation of $P_{50}$ shift [%] to number of erythrocytes/mm$^3$ in mice having received ITPP. It appears, based upon preliminary data, that an inverse relationship exists between the number of red blood cells and shift of their $P_{50}$ value. The basal value of the red blood cell count is restored, once $\Delta P_{50}$ becomes 0%, 12 days after ingestion of ITPP.

EXAMPLE 2

Induced Right Shift of the ODC in Mice (Injected Intraperitoneally)

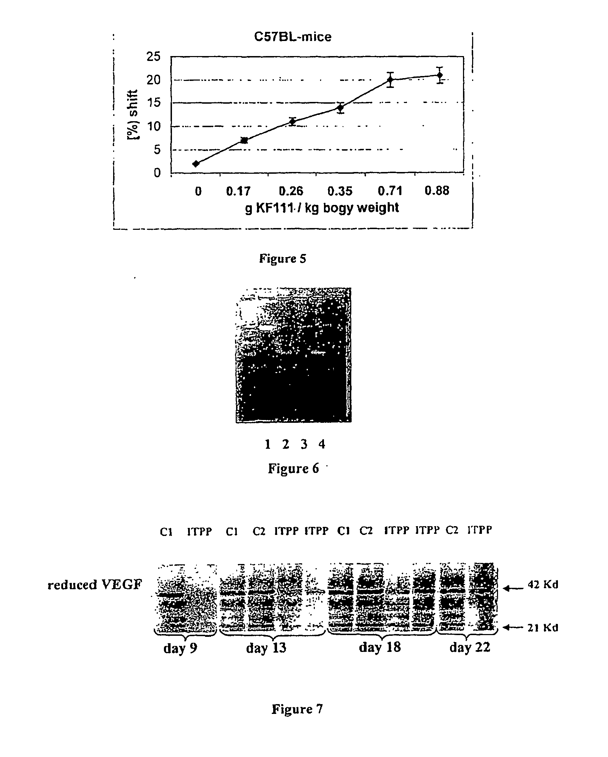

When ITPP (pH 7, 200μl) was injected intraperitoneally in mice, the $P_{50}$ values of circulating red blood cells were shifted up to 23%. Figure 5 demonstrates that ITPP was well tolerated by mice, up to a concentration of 150mM. The level of ions, such as sodium, potassium and calcium were normal after i.p. injection. Six (6) mice were each injected intraperitoneally with 45-150 mM (= 0.17-0.88 g/kg body weight) of ITPP. Means of % shift and standard deviation are shown in Figure 5.

The concentration dependence of the $P_{50}$ shifts induced by ITPP is an additional indication that this compound crosses the plasma membrane of the red blood cells.

EXAMPLE 3

Induced Right Shift of the OCD in Piglets (Intravenously Injected)

ITPP was also injected intravenously (IV) in piglets. A right shift of $P_{50}$ was observed, when the compound was injected at a 1g/kg body weight dose.

In order to check possible side effects of ITPP the level of calcium in the serum of the injected piglet was determined. A strong drop in the $Ca^{2+}$ concentration in the animal's blood immediately after infusion indicated the possibility that ITPP, with 3 dissociated phosphate groups binds $Ca^{2+}$, reducing thus its availability as free ion in the blood. One day after infusion the concentration of $Ca^{2+}$ in the piglets' blood was restored to the normal value. The results are shown in Table 1.

Table 1: $Ca^{2+}$ concentration in the piglet's circulation blood

| Sample taken | $Ca^{2+}$ conc. [mmol/L] |
|---|---|
| Before injection | 2.38 |
| 10 min after completion of injection | 1.73 |
| 24 hrs after injection | 2.36 |

Based upon this observation, a $CaCl_2$ (equimolar to ITPP) solution was injected with the TTPP solution, so that the dissociated phosphate groups of ITPP were saturated. None of the side effects observed previously occurred. The level of calcium remained constant and the $P_{50}$ shift was again approximately 20% of the basal value. The level of the other ions sodium and potassium was unchanged after IV injection of ITPP in piglets.

EXAMPLE 4

Effect of *in vivo* Lowering of Hemoglobin's Affinity for $O_2$ by ITPP on Intratumoral $PO_2$, Angiogenesis and Expression of VEGF mRNA

ITPP, when administered orally, intravenously, or intraperitoneally, inhibits angiogenesis in growing tumors by enhancing $PO_2$ in the forming tumors. Thirty (30) C57BL/6 mice received 20g/L of ITPP orally until the $P_{50}$ value showed a shift of at least 20% above the control value. All animals received then $1 \times 10^6$ Lewis Lung carcinoma (LLC) cells, injected in the dorsal cavity. At different time points, the VEGF mRNA were assayed by RT-PCR in the tumors growing in both groups of mice.

Tumor tissue samples were ground in a RIPA lysis buffer (1% Nonidet p-40 detergent, 50 mM Tris pH 8.0, 137 mM NaCl, 10% glycerol) supplemented with protease inhibitor cocktail (Roche, Reinach, Switzerland). After centrifugation (10 minutes, 4°C and 12,000 g), protein concentrations of tissue extracts were determined according to the Bradford method. Detergent soluble protein samples (10 mg) were size separated by SDS-PAGE in 10% acrylamide gels and transferred to nitrocellulose membrane (Protran BA 85, Schleicher and Schuell, Dassel, Germany). Membranes were blocked for 3 hours at room temperature in 10% skim milk in Tris buffer saline containing 0.1% Tween, before an overnight incubation at 4°C with rabbit polyclonal antibodies recognizing human, mouse and rat vascular endothelial growth factor (VEGF A-20, sc-152, Santa Cruz Biotechnology, Santa Cruz, California) at a dilution of 1:200. Membranes were then probed for primary antibody with anti-rabbit (1:16,000) peroxidase conjugates (Sigma-Aldrich, L'Isle d'Abeau Chesnes, France) for 60 minutes at room temperature. The resulting complexes were visualized by enhanced chemiluminescence autoradiography (Amersham Pharma Biotech, Orsay, France).

There was a difference in the level of mRNA of the VEGF gene in both groups. Figure 6 shows an agarose gel indicating the VEGF mRNA concentrations in tumors from control and ITPP drinking animals. The RT-PCR agarose gel assay of VEGF mRNAs from tumor tissue taken from 2 mice each on day 15 after inoculation of LLC cells (track 1: controls, track 2: ITPP treated animals) and day 30 after inoculation (track 3: control animals, track 4: ITPP treated animals). Figure 7 shows the Western blot assay of the expressed VEGF in tumors of control and ITPP-treated LLC tumor-bearing animals.

Quantification of the gel assays indicated a reduction by a factor of 10,000 of the amount of VEGF mRNAs detected in the tumors of animals having received ITPP, at day 9 and then, while differences remain between treated and untreated animals, they tend to decrease. This indicates that ITPP taken up by circulating red blood cells significantly increases tumor $PO_2$.

Having described the invention with reference to particular compositions, method for detection, and source of activity, and proposals of effectiveness, and the like, it will be apparent to those of skill in the art that it is not intended that the invention be limited by such illustrative embodiments or mechanisms, and that modifications can be made without departing from the scope or spirit of the invention, as defined by the appended claims. It is intended that all such obvious modifications and variations be included within the scope of the present invention as defined in the appended claims. The claims are meant to cover the claimed components and steps in any sequence that is effective to meet the objectives there intended, unless the context specifically indicates to the contrary.

REFERENCES

1. Fylaktakidou, K., Lehn, J.-M., Greferath, R, and Nicolau, C. (2005) Bioorg.Med.Chem. Lett, 15, 1605-1608.
2. Kim KJ, Li B, Winer J, Armanini M, Gillett N, Phillips HS, Ferrara N (1993) Nature 362, 841-844.
3. Kandel J, Bossy-Wetzel E, Radvanyi F, Klagsbrun M, Folkman J, Hanahan D (1991) Cell 66, 1095-1104.
4. O'Reilly MS, Boehm T, Shing Y, Fukai N, Vasios G, Lane WS, Flynn E, Birkhead JR, Olsen BR, Folkman J (1997) Cell 88, 277-285.
5. Good DJ, Polverini PJ, Rastinejad F, Le Beau MM, Lemons RS, Frazier WA, Bouck NP. (1990) Proc Natl Acad Sci USA 87, 6624-6628.
6. O'Reilly MS, Holmgren L, Shing Y, Chen C, Rosenthal RA, Moses M, Lane WS, Cao Y, Sage EH, Folkman J (1994) Cell 79, 315-328.
7. Chen C, Parangi S, Tolentino MJ, Folkman J. (1995) Cancer Res. 55, 4230-4233.
8. Ferrara N. (2002) Nat. Rev. Cancer 2, 795-803.
9. Ferrara N, Davis-Smyth T (1997) Endocr Rev. 18, 4-25.
10. Ferrara N, Gerber HP, LeCouter J. (2003) Nat Med. 9, 669-676.
11. Fontanini G, Vignati S, Boldrini L, Chine S, Silvestri V, Lucchi M, Mussi A, Angeletti CA, Bevilacqua G. (1997) Clin Cancer Res. 3, 861-865.
12. Dor Y, Porat R, Keshet E. (2001) Am J Physiol Cell Physiol. 280, C1367-1374.
13. Brizel DM, Scully SP, Harrelson JM, Layfield LJ, Bean JM, Prosnitz LR, Dewhirst MW (1996) Cancer Res. 56, 941-943.

**SUMMARY PARAGRAPHS:**

The invention relates to the following aspects and embodiments.

1. Inositol- tripyrophosphate for use in treating cancer in a human or animal.

2. Inositol- tripyrophosphate for use in enhancing oxygen delivery to hypoxic tumours in a human or animal.

3. Inositol- tripyrophosphate for use in enhancing radiation sensitivity of a tumour or tumours in a human or animal.

4. Inositol- tripyrophosphate for use in enhancing $PO_2$ in hypoxic tumours in a human or animal.

5. Inositol- tripyrophosphate for use in reducing the affinity of haemoglobin for oxygen in red blood cells in a human or animal.

6. The inositol- tripyrophosphate of any one of paragraphs 1 to 5, wherein the inositol-tripyrophosphate is for administration in a daily dose, a daily sub-dose, or any appropriate fraction thereof to the human or animal to inhibit the effects of angiogenesis.

7. The inositol- tripyrophosphate of any one of paragraphs 1 to 5, wherein the inositol-tripyrophosphate is for oral, parenteral, transdermal, topical, intravenous, intraperitoneal, subcutaneous, intramuscular, intradermal, ophthalmic, epidural, intratracheal, sublingual, buccal, rectal, vaginal, nasal or inhalation administration.

8. The inositol- tripyrophosphate of any one of paragraphs 1 to 5, wherein the inositol-tripyrophosphate is for administration in a composition comprising an additive selected from an antioxidant, a buffer, a bacteriostat, a liquid carrier, a solute, a suspending agent, a thickening agent, a flavoring agent, a gelatin, glycerin, a binder, a lubricant, an inert diluent, a preservative, a surface active agent, a dispersing agent, a biodegradable polymer, or any combination thereof.

9. The inositol- tripyrophosphate of any one of paragraphs 1 to 5, wherein the inositol-tripyrophosphate is for administration in the form of a tablet, a capsule, a lozenge, a cachet, a solution, a suspension, an emulsion, a powder, an aerosol, a suppository, a spray, a pastille, an ointment, a cream, a paste, a foam, a gel, a tampon, a pessary, a granule, a bolus, a mouthwash, or a transdermal patch.

10. The inositol- tripyrophosphate of paragraph 1, wherein the angiogenesis is associated with angiogenesis-dependent cancer.

11. The inositol- tripyrophosphate of paragraph 1, wherein the cancer is selected from breast cancer, prostrate cancer, renal cell cancer, brain cancer, ovarian cancer, colon cancer, bladder cancer, pancreatic cancer, stomach cancer, esophageal cancer, cutaneous melanoma, liver cancer, lung cancer, testicular cancer, kidney cancer, bladder cancer, cervical cancer, lymphoma, parathyroid cancer, penile cancer, rectal cancer, small intestine cancer, thyroid cancer, uterine cancer, Hodgkin's lymphoma, lip and oral cancer, skin cancer, leukemia or multiple myeloma.

12. A pharmaceutical preparation comprising inositol-tripyrophosphate and a pharmaceutically acceptable carrier, excipient or diluent.

13. Inositol- tripyrophosphate for use in treating Alzheimer's disease in a human or animal.

14. Inositol- tripyrophosphate for use in treating stroke or osteoporosis.

15. Use of inositol- tripyrophosphate in the manufacture of a medicament for:

(i) treating cancer in a human or animal;

(ii) enhancing oxygen delivery to hypoxic tumours in a human or animal;

(iii) enhancing radiation sensitivity of a tumour or tumours in a human or animal;

(iv) enhancing $PO_2$ in hypoxic tumours in a human or animal;

(v) reducing the affinity of haemoglobin for oxygen in red blood cells in a human or animal;

(vi) treating Alzheimer's disease in a human or animal; and/or

(vii) treating stroke or osteoporosis.

**Claims**

1. Inositol- tripyrophosphate for use in treating Alzheimer's disease in a human or animal.

2. Inositol- tripyrophosphate for use in treating stroke or osteoporosis.

3. The inositol- tripyrophosphate of Claim 1 or Claim 2, wherein the inositol-tripyrophosphate is for administration in a daily dose, a daily sub-dose, or any appropriate fraction thereof to the human or animal to inhibit the effects of angiogenesis.

4. The inositol- tripyrophosphate of Claim 1 or Claim 2, wherein the inositol-tripyrophosphate is for oral, parenteral, transdermal, topical, intravenous, intraperitoneal, subcutaneous, intramuscular, intradermal, ophthalmic, epidural, intratracheal, sublingual, buccal, rectal, vaginal, nasal or inhalation administration.

5. The inositol- tripyrophosphate of Claim 1 or Claim 2, wherein the inositol-tripyrophosphate is for administration in a composition comprising an additive selected from an antioxidant, a buffer, a bacteriostat, a liquid carrier, a solute, a suspending agent, a thickening agent, a flavoring agent, a gelatin, glycerin, a binder, a lubricant, an inert diluent, a preservative, a surface active agent, a dispersing agent, a biodegradable polymer, or any combination thereof.

6. The inositol- tripyrophosphate of Claim 1 or Claim 2, wherein the inositol-tripyrophosphate is for administration in the form of a tablet, a capsule, a lozenge, a cachet, a solution, a suspension, an emulsion, a powder, an aerosol, a suppository, a spray, a pastille, an ointment, a cream, a paste, a foam, a gel, a tampon, a pessary, a granule, a bolus, a mouthwash, or a transdermal patch.

7. Use of inositol- tripyrophosphate in the manufacture of a medicament for:

    (i) treating Alzheimer's disease in a human or animal; and/or
    (ii) treating stroke or osteoporosis.

Figure 1

Figure 2

Figure 3

Preliminary data indicating the relationship between RBC count and P50 value

Figure 4

**Figure 5**

1  2  3  4

**Figure 6**

**Figure 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4192869 A
- US 4321259 A
- US 4473563 A, Nicolau
- US 4752586 A
- US 4652449 A, Ropars
- US 4874690 A
- US 5043261 A, Goodrich
- US 4478824 A
- US 4931276 A, Franco
- US 5612207 A

**Non-patent literature cited in the description**

- **R. E. Benesch et al.** *Biochemistry,* 1977, vol. 16, 2594-2597
- *J. Biol. Chem.,* 1975, vol. 250, 7093-7098
- **Gersonde et al.** Modification of the Oxygen Affinity of Intracellular Haemoglobin by Incorporation of Polyphosphates into Intact Red Blood Cells and Enhanced 02 Release in the Capillary System. *Biblthca. Haemat,* 1980, 81-92
- **Gersonde et al.** Enhancement of the O2 Release Capacity and of the Bohr-Effect of Human Red Blood Cells after Incorporation of Inositol Hexaphosphate by Fusion with Effector-Containing Lipid Vesicles. *Origins of Cooperative Binding of Hemoglobin,* 1982
- **Weiner.** Right Shifting of Hb-O2 Dissociation in Viable Red Cells by Liposomal Technique. *Biology of the Cell,* 1983, vol. 47
- **Nicolau et al.** Incorporation of Allosteric Effectors of Hemoglobin in Red Blood Cells. Physiologic Effects. *Biblthca. Haemat.,* 1985, 92-107
- **Mouneimne et al.** Stable rightward shifts of the oxyhemoglobin dissociation curve induced by encapsulation of inositol hexaphosphate in red blood cells using electroporation. *FEBS,* 1990, vol. 275 (1, 2), 117-120
- **Folkman.** *New Eng. J. Med.,* 1971, vol. 285, 1182-86
- **Vagnucci et al.** Alzheimer's disease and angiogenesis. *Lancet,* 15 February 2003, vol. 361 (9357), 605-608
- **Pogue et al.** Angiogenic signaling in Alzheimer's disease. *Neuroreport,* 28 June 2004, vol. 15 (9), 1507-1510
- **Ishii et al.** *J. Nucl. Med.,* July 1996, vol. 37 (7), 1159-65
- **Brem et al.** *J. Neurosurg,* 1991, vol. 74, 441-446
- **Fylaktakidou, K. ; Lehn, J.-M. ; Greferath, R ; Nicolau, C.** *Bioorg.Med.Chem. Lett,* 2005, vol. 15, 1605-1608
- **Kim KJ ; Li B ; Winer J ; Armanini M ; Gillett N ; Phillips HS ; Ferrara N.** *Nature,* 1993, vol. 362, 841-844
- **Kandel J ; Bossy-Wetzel E ; Radvanyi F ; Klagsbrun M ; Folkman J ; Hanahan D.** *Cell,* 1991, vol. 66, 1095-1104
- **O'Reilly MS ; Boehm T ; Shing Y ; Fukai N ; Vasios G ; Lane WS ; Flynn E ; Birkhead JR ; Olsen BR ; Folkman J.** *Cell,* 1997, vol. 88, 277-285
- **Good DJ ; Polverini PJ ; Rastinejad F ; Le Beau MM ; Lemons RS ; Frazier WA ; Bouck NP.** *Proc Natl Acad Sci USA,* 1990, vol. 87, 6624-6628
- **O'Reilly MS ; Holmgren L ; Shing Y ; Chen C ; Rosenthal RA ; Moses M ; Lane WS ; Cao Y ; Sage EH ; Folkman J.** *Cell,* 1994, vol. 79, 315-328
- **Chen C ; Parangi S ; Tolentino MJ ; Folkman J.** *Cancer Res.,* 1995, vol. 55, 4230-4233
- **Ferrara N.** *Nat. Rev. Cancer,* 2002, vol. 2, 795-803
- **Ferrara N ; Davis-Smyth T.** *Endocr Rev.,* 1997, vol. 18, 4-25
- **Ferrara N ; Gerber HP ; LeCouter J.** *Nat Med.,* 2003, vol. 9, 669-676
- **Fontanini G ; Vignati S ; Boldrini L ; Chine S ; Silvestri V ; Lucchi M ; Mussi A ; Angeletti CA ; Bevilacqua G.** *Clin Cancer Res.,* 1997, vol. 3, 861-865
- **Dor Y ; Porat R ; Keshet E.** *Am J Physiol Cell Physiol.,* 2001, vol. 280, C1367-1374
- **Brizel DM ; Scully SP ; Harrelson JM ; Layfield LJ ; Bean JM ; Prosnitz LR ; Dewhirst MW.** *Cancer Res.,* 1996, vol. 56, 941-943